# EUROPEAN PATENT APPLICATION

(11) **EP 0 828 006 A2**
(43) Date of publication of application: **11.03.1998**
(21) Application number: 97870134.0
(22) Date of filing: 10.09.1997
(51) Int. Cl.: C12N 15/86, C12N 9/90, C12N 5/10, A61K 48/00, C12N 15/00, A01K 67/027

(54) **Use of cis- and/or trans-elements to enhance the efficiency of parvoviral vector transduction, integration and/or transgene expression**

(30) Priority: 10.09.1996 US 25734 P
(71) Applicant: VRIJE UNIVERSITEIT BRUSSEL, 1050 Brussel (BE)
(72) Inventor: Peters, Elizabeth, 3140 Keerbergen (BE); Tenenbaum, Liliane, 1090 Brussels (BE)
(74) Representative: Van Malderen, Joelle

(57) **Abstract**

The present invention is related to a method for increasing the efficiency of the integration of a parvoviral vector into a cell genome and the expression of genetic sequence carried by said vector, wherein this increase results from the insertion or the addition of non-parvoviral cis- and/or trans-elements made of nucleic acid sequence(s) and/or amino acid sequence(s) (encoded by said sequence(s)), into a parvoviral vector.

## Description

### Field of the invention

The present invention is related to the use of cis- and/or trans-elements to improve the transduction and/or integration of parvoviral vectors as well as the expression of the transgene carried by said vector.

The present invention is also related to a new vector having improved transduction and/or integration properties, the cell and animal containing said vector and a pharmaceutical composition comprising said vector and/or said cell.

The present invention is also related to the use of said products for gene transfer especially in the fields of gene therapy and of the immortalisation of cells.

### Background of the invention and state of the art

Vectors presently used for gene therapy include adenovirus-based vectors, retrovirus-based vectors and adeno-associated based vectors.

All these vectors present drawbacks especially :
i) for the treatment of chronic diseases for which sustained long-term expression of the therapeutic gene is needed;
ii) for cell immortalisation for which high level and long-term expression of the immortalizing gene is needed.

Adenoviral vectors allow a high level of expression of the transgene. However they do not integrate in the cellular genome and therefore are diluted when the cell divides and they contain viral genes whose products can provoke an immune reaction toward the transduced cells.

Retroviral vectors integrate in the cellular genome. However, they infect only actively dividing cells, their titer is too low to use them for direct in vivo gene therapy, and they infect only some cell types bearing a specific receptor.

The human defective parvovirus adeno-associated virus (AAV) infects a wide range of host tissues and species. AAV can be propagated either in a latent state as an integrated provirus or by lytic infection in the presence of a co-infecting helper virus (Berns & Bohenzky, 1987). In the absence of helper virus, AAV genome integrates into the genomic DNA of infected cells preferentially into a specific site (called S1) in human chromosome 19 (Kotin et al.,1990; review: Samulski, 1993). This event appears to be triggered by the AAV Rep proteins which form a complex with AAV inverted terminal repeats (ITRs) and the S1 site (Weitzman et al., 1994). Consistently, AAV vectors containing the ITRs and the rep gene integrate into S1 (Shelling and Smith, 1994) whereas vectors containing only the ITRs, although proficient for integration do not target chromosome 19 (review: Flotte and Carter, 1995).

The limitation to the use of AAV vectors for gene therapy resides in their low transduction efficiency. Indeed, several thousands of viral particles are necessary to transduce a large proportion of the cells in cultures of established cell lines (Samulski et al., 1989; Fisher et al. 1996; Ferrari et al, 1996, Chiorini et al., 1995). Furthermore, to evaluate the potentialities of AAV vectors for human gene therapy, the transduction efficiency of primary cells directly cultured from patient tissue must be evaluated. Few studies addressed this question. However, it has been shown in some cases that AAV vectors transduce primary cells with a much lower efficiency than established cell lines (Halbert et al. 1995). Several studies suggested that AAV vectors can transduce non-proliferative cells (Podsakoff et al 1994, Kaplitt et al, 1994, Ali et al, 1996, Alexander et al., 1996, Zhou et al., 1994). However, GO cells are transduced at a much lower efficiency than S-phase cells (Russel et al 1994).

The transduction efficiency can be drastically enhanced by several means: providing Ad E4 and El genes in trans- (Ferrari et al, 1996, Fisher et al., 1996), treating cells with DNA damaging agents (Ferrari et al. 1996, Alexander et al. 1994, Alexander et al., 1996) or other non toxic treatments known to block cells in the S-phase and to induce unscheduled DNA synthesis (Russel, 1995). Conversion of single-stranded input DNA to double-stranded monomeric replication intermediates which are transcriptionally active seems to be responsible for the enhanced transduction efficiency. In the presence of the helper, the recruitment of the recombinant AAV (rAAV) genomes in the intranuclear domains in which adenovirus replicates further supports this hypothesis (Weitzman et al. 1996). The converted (double-stranded) rAAV genomes appearing in the presence of El and E4 gene products were present as episomes and whether they can later on integrate was not looked for in these studies. Treatments that stimulate transduction by rAAV also stimulate replication of wild-type AAV (Yakobson et al., 1989, Shlehöfer 1986, Yalkinoglu 1988, Yakobson 1987). These situations in which transduction efficiency is enhanced by stimulating second-strand synthesis thus probably mimic the first stages of the lytic pathway rather than the latent pathway.

The capacity of integration is however important for exploiting AAV as a vector for gene therapy of chronic diseases for which prolonged expression of the therapeutic transgene is required. Vectors retaining the rep gene (Hermonat & Muzyczka, 1984, Tratschin, 1985) integrate at chromosome 19 but their transduction efficiency is much lower than that of rep⁻ vectors (Mc Laughlin et al, 1988). Rep proteins are involved in various aspects of AAV life cycle including replication, integration, rescue and transcription control. This pleiotropy is reflected by the existence of functional domains, which have been partially identified by mutagenesis (Mc Carty et al., 1992; Owens et al., 1993; Yang et al., 1992; Weitzmann et al., 1996). Rep proteins also influence the metabolism of transformed cells through growth retardation (Bantel-Schaal and Stöhr, 1992; Winocour et al., 1988, 1992) and transcription inhibition (Hermonat). The inhibition of heterologous gene expression by Rep proteins (Labow et al, 1986, Mendelson et al 1988) is probably responsible for the reduced transduction frequency of rep⁺ vectors.

### Aims of the invention

The present invention aims to provide a vector which does not present the drawbacks of the products of the state of the art.

A main aim of the invention is to provide a vector which has improved transduction and/or integration properties.

Another aim of the present invention is to provide a vector which may be used for gene transfer into various cells including non dividing cells.

A further aim of the invention is to provide a vector which may be used for the immortalization of non dividing cells.

A last aim of the present invention is to provide a pharmaceutical composition which comprises said vector and said genetically modified cells for use in genetic therapy and/or for the immortalization of cells.

### Summary of the invention

The inventors have hypothetised that both the high infectivity and/or integration capacities of wild-type AAV can be exploited for the development of vectors for efficient and sustained gene transfer by combining parvoviral palindromic extremities used as minimal core vector- with cis- and/or trans-acting sequence(s) providing both transcription activating and recombination (integration) functions.

The present invention concerns a method for increasing the efficiency of
- the transduction of a parvoviral vector,
- the integration of said vector into a cell genome,
- the expression of genetic sequences (including foreign genetic sequences) carried by said vector,
wherein this increase results from the insertion and/or the addition of non-parvoviral cis- and/or trans-elements made of nucleic acid sequence(s) and/or amino acid sequence(s) (encoded by said nucleic acid sequence(s)) into said ("derived") parvoviral vector.

Therefore, the present invention is also related to a vector comprising, associated with a parvovirus or a portion thereof, a transduction and/or integration enhancing nucleotide and/or amino-acid sequence(s) from another virus and/or a cellular factor.

Vectors according to the invention may comprise the parvovirus palindromic extremities or a portion thereof. Said parvovirus is derived from human, mammal, avian viruses belonging to the adeno-associated viruses or derived from human or mammal autonomous parvoviruses.

The parvoviruses are viruses well-known by the man skilled in the art and are for instance described in the document Handbook of parvoviruses (1990, volume 2, CRC Press Inc., Boca Raton, Florida).

The autonomous parvoviruses are small viruses with no envelope whose genome consisting of linear-single-stranded DNA of about 5 kd. Such viruses are for instance the parvovirus H1, the fibrotropic parvovirus of "minute virus of Mice" (MVMp or MVMi) or the parvovirus Lu III.

In the vector according to the invention the transduction and integration enhancing element is a nucleotidic and/or amino-acid sequence having transcription activation and/or recombination/integration enhancing activity. The transcription activation element may be a viral transcription activator may be e.g. the transcription activation domain of papovavirus large T antigen, adenovirus E1A or E1B, human papilloma virus E6 or E7, non-structural gene or gene product(s) encoded peptides or a portion thereof from autonomous parvoviruses.

The recombination enhancing element may be the recombination enhancing domain of papovavirus large T antigen, retrovirus integrases (e.g. HIV integrase), yeast recombination genes or gene products (e.g. RAD51) bacterial recombination genes or gene products (e.g. E.coli recA) or a portion thereof.

In the vector according to the invention, the transduction and integration enhancing nucleotidic and/or amino-acid sequence is preferably the SV40 early operon or a portion thereof. The vector according to the invention may comprise the complete nucleotide sequence of the SV40 early operon (map position 270-0/5243-2533), a portion thereof, a mutant thereof or encoded peptide(s) by said nucleotide sequence or a portion thereof or a mutant thereof, if said portion is a transduction and integration enhancing sequence. Preferably, the nucleotide sequence comprises the sequence encoding the functions of replication elongation, DNA helicase and/or transcription activation. According to a preferred embodiment of the present invention, the nucleotide sequence of the early operon lacks part or all of the nucleotide sequence corresponding to the amino acid 105 to 115 of the large T antigen, or the amino acid sequence lacks the amino acid 105 to 115 of the large T antigen.

In the vector according to the invention, the nucleotide and/or amino acid sequence of the SV40 early operon are also variants of said sequence (which comprises mutation or deletion of the nucleotide and/or amino acid sequence) which do not reduce the transduction and/or integration enhancing properties of the orginal sequence.

According to the invention, the terms "transduction and/or integration enhancing sequence" means that the vector according to the invention is able to transduce a target cell population at a frequency higher than 1 transductant per 500 input vector genomes in the absence of selective pressure.

The transduction frequency could be measured by various methods. According to the state of the art, one infectious unit is defined as one replicative center as measured by in situ focus hybridization assay (Yakobson et al., 1989). One infectious unit (I.U) corresponds to roughly 100 viral genomes (single-stranded DNA molecules) as evaluated in southern blots by comparing with a given amount of pTR-TtsA58 plasmidic DNA using a labelled KpnI-BamHI SV40 fragment. Therefore, by using the AAV/TtsA58 viral vector, when 2 10⁴ cells are infected by 10⁵ I.U. or 10⁷ viral genomes, more than 50% of the cells express the T protein and more than 90% of the cells express the T mRNA. In other words, 5 I.U or 500 viral genomes are required to produce one transductant (1 cell harbouring a transcriptionally active viral genome). By using an AAV vector expressing the gfp as well as the neo reporter genes (Zolotukhin et al., 1996), when 2 10⁴ cells are infected by 10⁵ I.U. OR 10⁷ viral genomes, 1 to 10 gfp-positive are observed (i.e. 0.05 to 0.5 %) and 1 to 5 % of the cells efficiently express the neo mRNA. In other words, 500 I.U or 510⁴ viral genomes are required to produce one transductant (1 cell harbouring a transcriptionally active viral genome).

Preferably, in the vector according to the invention, the nucleotide or the amino acid sequence(s) of the SV40 early operon or a portion thereof is associated with a parvovirus or a portion thereof. This means that the nucleotide sequence(s) of said operon could be integrated into the nucleotide sequence(s) of the parvovirus or a portion thereof (for instance between the palindromic extremities of said parvovirus).

According to another embodiment of the present invention, the amino acid sequence (s) (the peptide(s) encoded by SV40 early operon) could also be associated with said parvovirus DNA sequence(s) as a derived (non viral) parvoviral vector.

The vector according to the invention may also comprise a foreign nucleotidic and/or amino acid sequence which could be used for the genetic modification of target cells.

Another aspect of the present invention also concerns the cells which are genetically-modified by the vector according to the invention. The genetically-modified cell according to the invention is preferably a cell chosen among the group consisting of glial cells, neuronal cells, fibroblasts Rematopoietic (including stem cells) and/or pituitary cells.

The genetically modified cells according to the invention are non human or human cells with the proviso that when they are human cells, they are only somatic cells.

The present invention is also related to a pharmaceutical composition comprising the vector and/or the cell(s) according to the invention and a pharmaceutically acceptable carrier.

The present invention is related to the use of the pharmaceutical composition according to the invention in the preparation of a medicament, in the prevention and/or treatment of a disease preferably chosen among the group consisting of neurodegenerative diseases, cancer (in particular glioma) or genetic diseases.

Another aspect of the present invention is related to the use of the vector according to the invention for the preparation of a medicament in the treatment and/or prevention of somatic cells infections by viral agents such as HIV, HTLV, HSV, HPV; ..., bacterial agents or by other intra-cellular infectious parasites (such as plasmodium). The terms "treatment and/or prevention" mean a decrease of the symptom induced by said agents, the elimination or the inhibition of the causative agent, the prevention of somatic cell infection or disorder in subjects suffering from said symptoms.

A last aspect of the present invention is related to the use of the vector according to the invention for the immortalisation of cells, preferably human somatic cells such as a cell of the endocrine, nervous and/or immune system.

### Brief description of the drawings

- The figure 1: represents the structure of the pTR-TtsA58 plasmid.
- The figure 2: represents the Titration of the AAV/TtsA58 recombinant viral stock.
HeLa cells were co-infected with adenovirus (100 pfu/cell), wild-type AAV (200 IU/cell) and 10 ml (A) or 50 ml (B) of a 1:100 dilution of the viral stock. HeLa cells were co- infected with wild-type AAV (200 IU/cell) and 10 ml of the undiluted AAV/TtsA58 stock (C). Twenty-eight hours post-infection, the cell monolayer was transferred to nitrocellulose filters. Filters were hybridized as described in Material and Methods.

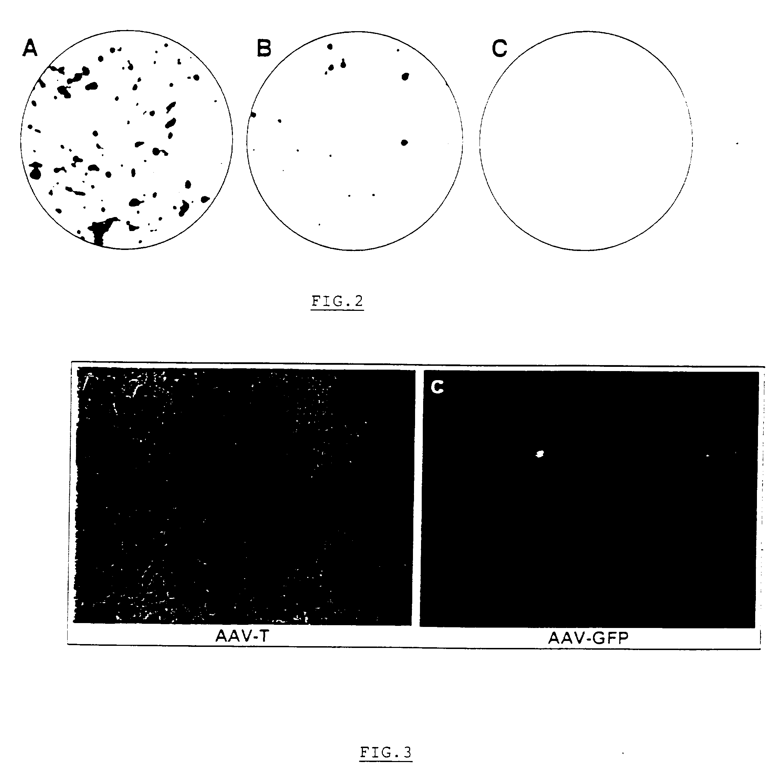

- The figure 3: represents the Comparison of the transduction efficiency of AAV/TtsA58 and AAV/gfp.
HeLa cells were infected with AAV/TtsA58 (A, B) or AAV/gfp (C, D) at a multiplicity of 10 IU per cell. After overnight incubation at 37 °C, AAV/TtsA58 infected cells were incubated at 33 °C (A) or 38 °C (B) for 2 days. T antigen expression was revealed by immunocytochemistry. Cells infected by AAV/gfp were photographed under U.V. light (C, D) combines with visible light (D) to visualize the non-labelled cells.

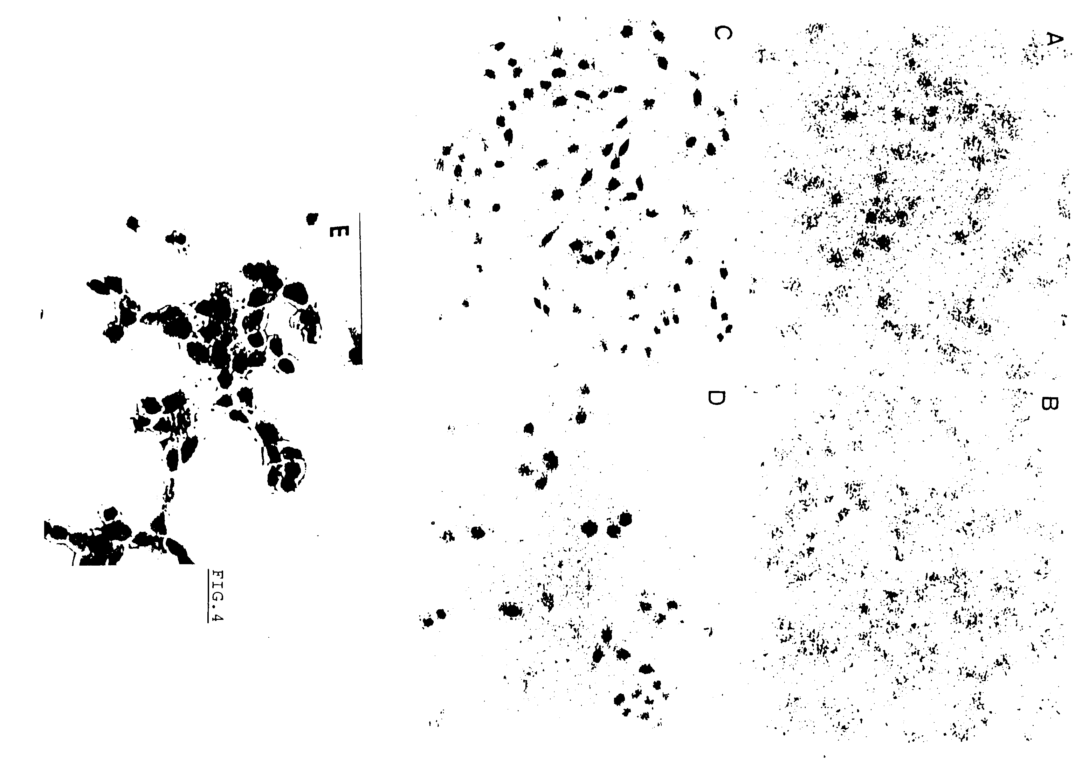

- The figure 4: represents the measure of T antigen and neo mRNA expression in HeLa cells evidenced by *in situ* hybridization. HeLa cells were infected with AAV/TtsA58 (A, B) or AAV/gfp (C, D) at a multiplicity of 10 IU per cell. After virus adsorption at 37 °C, AAVTtsA58-infected cells were transferred at 33 °C or 38 °C.
A. AAV/TtsA58 infected cells at 33 °C, 5 days post-infection;
B. AAV/TtsA58 infected cells at 38 °C, 5 days post-infection;
C. AAV/TtsA58 infected cells at 33 °C, 2 hours post-infection;
D. AAV/TtsA58 infected cells at 33 °C, 5 days post-infection; slides were treated with RNAse at a concentration of 50 mg/ml before hybridization.
E. AAV/gfp infected cells 5 days after infection.

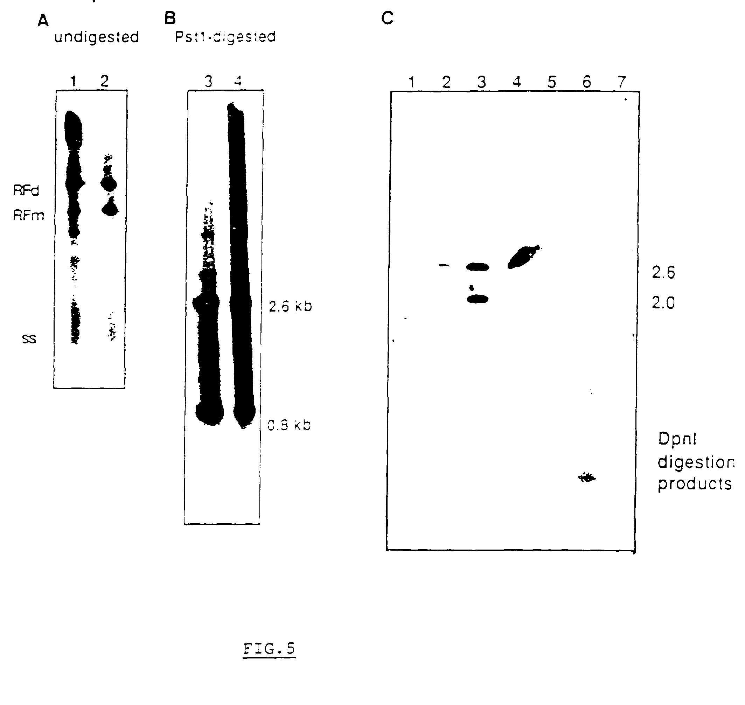

- The figure 5: represents the production of the typical AAV-like replication forms after infection by AAV/TtsA58 in the presence of wild-type AAV and adenovirus as well as in the presence of adenovirus only.
HeLa cells were co-infected with AAV/TtsA58 at a multiplicity of 10 IU per cell and with adenovirus and wild-type AAV at a multiplicity of 100 IU per cell (panels A and B) or with adenovirus only (panel C). After virus adsorption at 37 °C, cells were transferred at 38 °C (lane 1) or 33 °C (lane 2). Low molecular weight DNA was extracted 48 hrs post-infection, digested with DpnI (panels A, C) or with DpnI and PstI (panel B) and electrophoresed through a 1% agarose gel. The blot was hybridized with a ³²P-labelled SV40 fragmented.

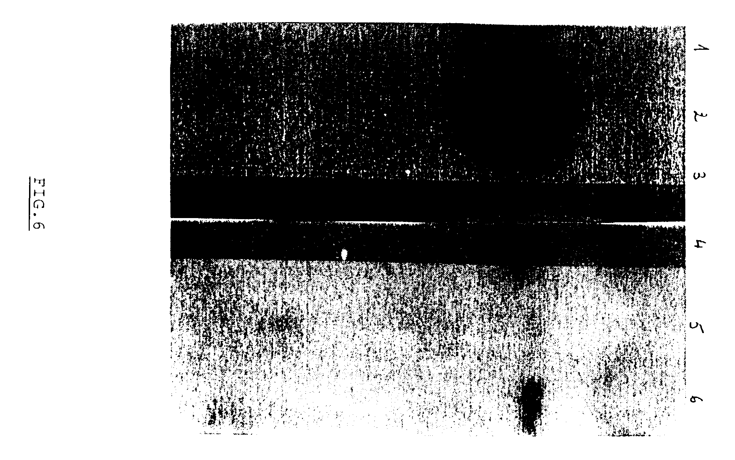

- The Figure 6: represents the functionality of tsA58 T antigen and SV40 origin of replication in the pTR/TtsA58 plasmid at 33 °C in permissive monkey cells.
CV1 monkey cells were either infected by SV40tsA58 virus (lanes 1&4) or transfected by SV40tsA58 viral DNA (lanes 2&5) or pTR-TtsA58 DNA (lanes 3&6). Four hours (lanes 1, 2, 3) or 5 days (lanes 4, 5, 6) after infection or transfection, low molecular weight DNA was extracted and electrophoresed through a 1 % agarose gel. The blot was hybridized with a ³²P-labelled SV40 fragment.

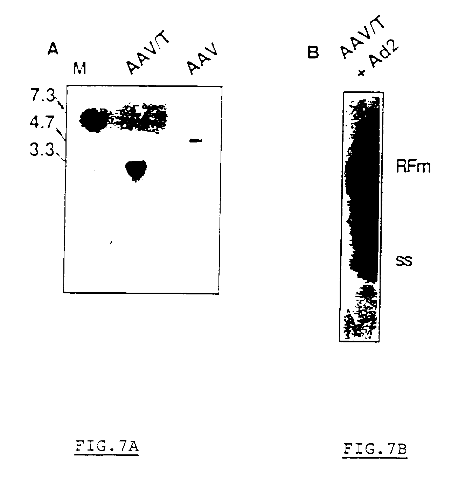

- The figure 7: represents the absence of conversion of AAV/TtsA58 input DNA to double-stranded DNA in the absence of adenovirus. HeLa cells were infected with AAV/TtsA58 at a multiplicity of 10 IU per cell. After virus adsorption at 37 °C, cells were transferred at 33 °C. Low molecular weight DNA was extracted 24 hrs post-infection and electrophoresed through a 1.2% alkaline agarose gel. M: marker, 7.6 kb pTR-Ttsa58 plasmid, AAV: decapsidated viral AAV DNA. Blots were hybridized with ³²P-labelled SV40 or AAV fragments.

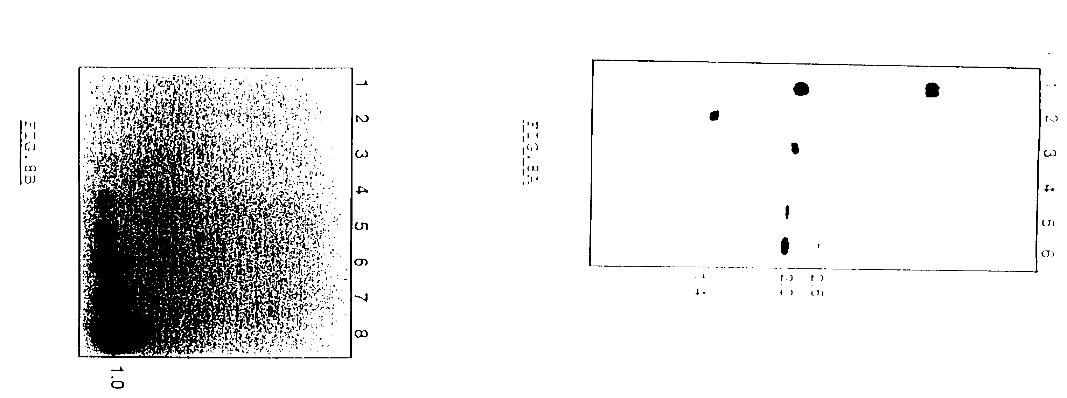

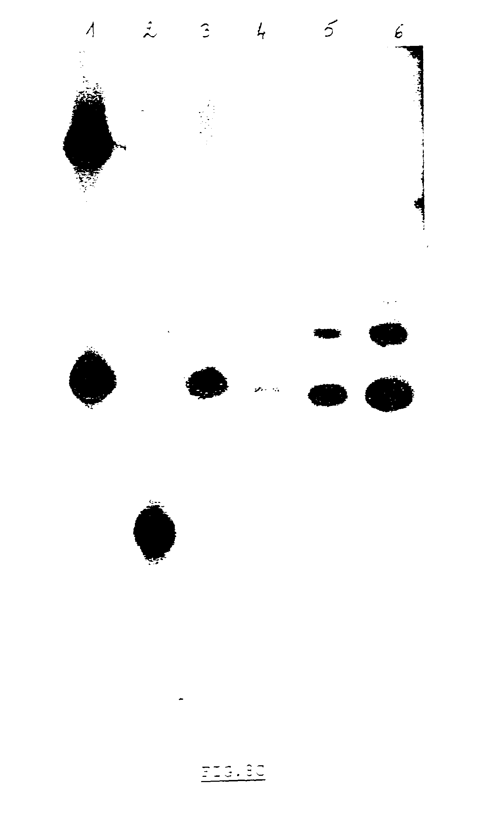

- The figure 8: represents the association of AAV/TtsA58 DNA with cellular DNA.
HeLa cells were infected with AAV/TtsA58 at a multiplicity of 5 IU per cell. After overnight incubation at 37 °C, infected cells were incubated at 38 °C or 33 °C for 5 days. Genomic DNA (panel A) and low molecular weight (viral) DNA (panel) were extracted and electrophoresed through a 1% agarose gel (panel A).
Panel A: lane 1: Cos1 (SV40 ori⁻ -transformed CV1 simian cells);
lane 2: AAV/TtsA58 infected HeLa cells at 38 °C;
lane 3: AAV/TtsA58 infected HeLa cells at 33 °C;
lanes 4 to 6: marker PvuII digested pTR-TtsA58 DNA, corresponding to 0.5 (lane 4), 1 (lane 5), 2 (lane 6) copies of the viral genome per cell.
Panel B: lanes 1 to 3: marker PvuII digested pTR-TtsA58 DNA, corresponding to 0.5 (lane 1), 1 (lane 2), 2 (lane 2) and 5 (lane 3) copies of the viral genome per cell;
lanes 4&6: AAV/TtsA58 infected HeLa cells at 38 °C;
lanes 5&7: AAV/TtsA58 infected HeLa cells at 33 °C, 2 days (lanes 4&5) or 5 days (lanes 6&7) after infection.
Panel C : represents the lack of association of AAV/CAT-Neo recombinant virus with cellular DNA.
HeLa cells were infected with AAV/CAT-Neo at a multiplicity of 5 IU per cell. After 5 days incubation at 37 °C, genomic DNA was extracted, digested with PstI (lane 1) or SmaI (lane 2) and electrophoresed through a 1 % agarose gel. Lanes 3 to 8 : PstI-digested pTR-CAT-Neo plasmid corresponding respectively to 1, 2, 5, 10, 20 and 50 copies per genome.

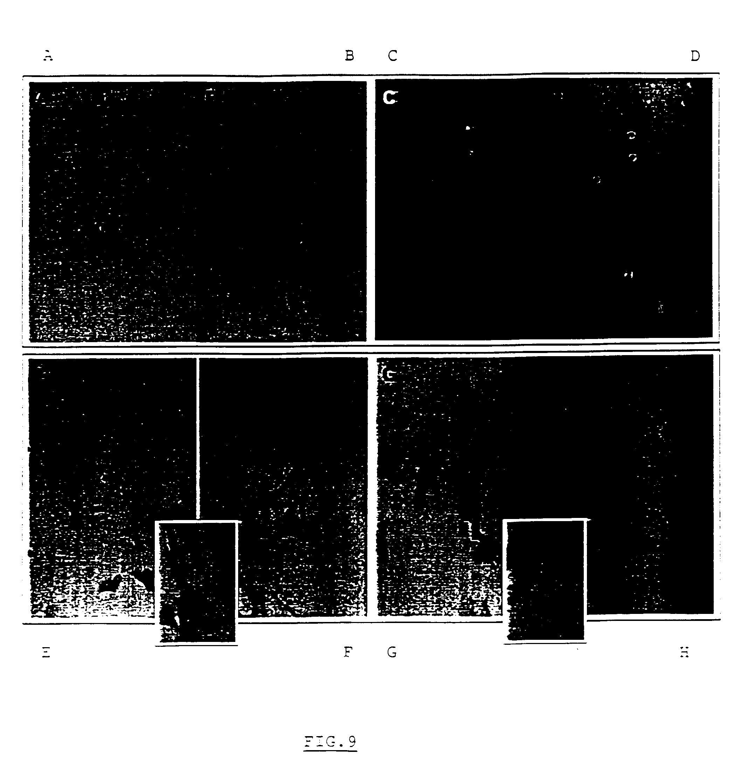

- The figure 9: represents the Expression of SV40 T antigen in infected primary cells evidenced by immunocytochemistry.
Cells were infected with AAV/TtsA58 at a multiplicity of 5 IU per cell. After overnight incubation at 37 °C, the cells were transferred at 33 °C or 38 °C.
C, D: pituitary adenomas;
A, B: 2 days after infection;
C, D: 6 weeks after infection;
A, C, D: at 33 °C;
B: at 37 °C;
G, H: oligodendroglioma cells;
E, F: cultured in serum-supplemented medium;
G, H: cultured in serum-free medium;
E, G: infected cells;
F, H: non-infected cells.
Proliferative behaviour as evidenced by ³H-thymidine incorporation is shown.

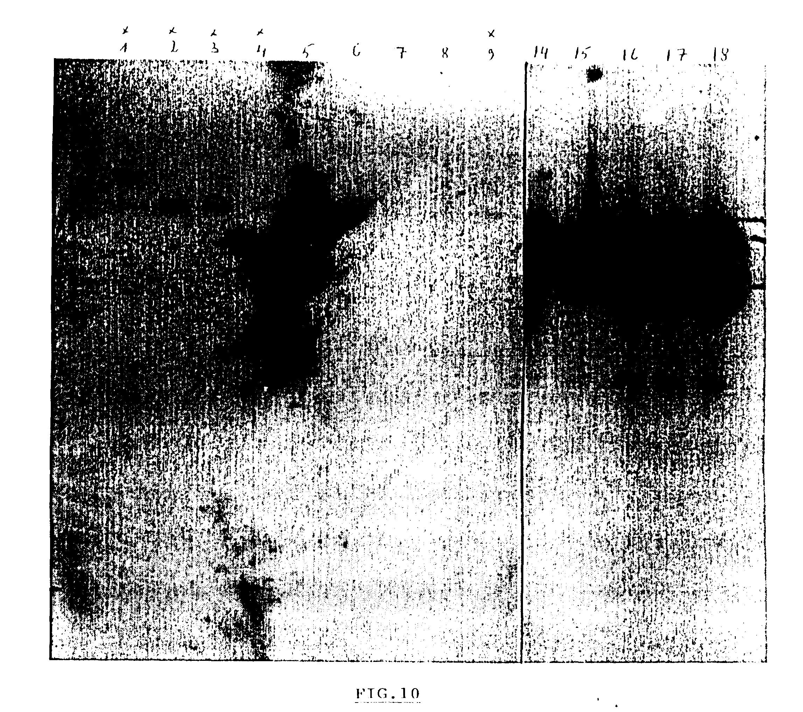

- The Figure 10: represents the association of AAV/TtsA58 DNA with cellular DNA in cloned populations of AAV/TtsA58-infected cells cultured at 33 °C for 25 generations (3 months in culture) after infection.
Panel A: Lanes 1 to 9: 20 µg (corresponding to approximately 2 10⁶ cells) of PvuII-digested genomic DNA extracted from 9 independent cellular clones;
Lanes 14 to 18: marker PvuII-digested pTR-TtsA58 DNA corresponding to 1, 2, 3, 4 and 5 copies of the viral genome pet cell. The blot was hybridized with a ³²P-labelled SV40 fragment.

### Description of a preferred embodiment of the present invention.

The inventors have discovered unexpectedly that an AAV/SV40 hybrid vector expressing the tsA58 thermosensitive mutant of SV40 large T antigen as well as small t antigen (AAV/T) transduced human cells of various tissue-origins at high efficiency. The enhanced transduction as compared to AAV vectors was not due to an enhanced conversion into double-stranded episomal DNA. In contrast, AAV/T sequences were found to be associated with cellular DNA at high frequency. This vector thus mimics the integrative behaviour of AAV in the latent state while avoiding the inhibition of transgene expression by Rep proteins. The transduction efficiency was identical at 33 °C (active tsA58 T antigen) and 38 °C (inactivated tsA58 T antigen) suggesting that only a subset of T antigen functions are necessary for the observed enhancement of AAV vectors-mediated transduction. Furthermore, AAV/T efficiently transduced non-dividing primary human oligodendroglioma and pituitary adenoma cells.

pTR (from S. Zolotukhin and N. Muzyczka, University of Florida, Gainesville) contains AAV inverted terminal repeats (map position 1-145 in AAV map, gene bank accession number j01901) cloned in pBR322.

pTR-UF2 (from S. Zolotukhin and N. Muzyczka) has been described (Zolotukhin et al., 1996) and contains the "humanised" gfp ("green fluorescent protein") gene under the control of the CMV promoter and the neo gene under the control of the HSV-TK promoter.

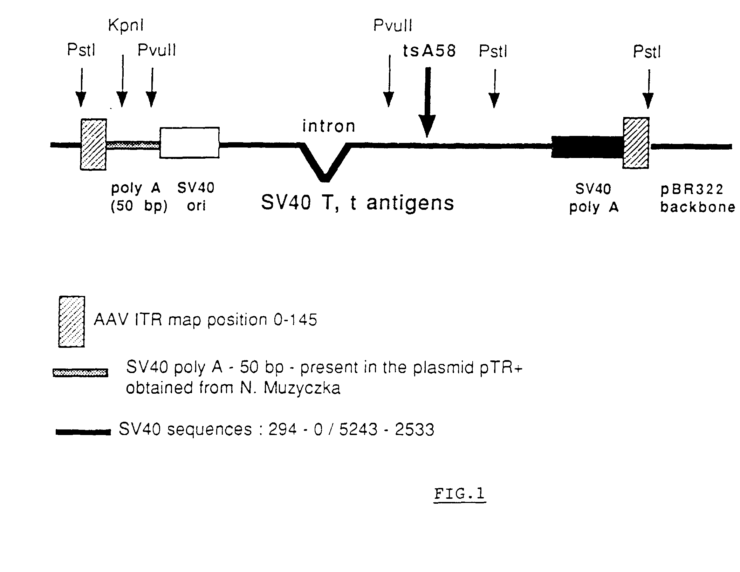

pTR-TtsA58 was constructed by inserting the KpnI-BamHI 3 kb fragment from SV40 tsA58 virus, which contains the SV40 early genes (tsA58 mutated large T and small t antigens), replication origin and polyadenylation signal, into pTR (Fig. 1).

pTR/CAT-Neo was constructed by replacing the gfp expression cassette in PTRUF2 by the CAT expression cassette (BgIII-SalI fragment) from the pCAT-control vector from Promega.

The plasmid pIM45 contains AAV coding regions (map position 190 to 4489).

Plasmids were isolated and propagated in the JC8111 bacterial strain (Boissy & Astell, 1986) and purified using QUIAGEN columns according to the protocol of the manufacturer, followed by polyethylene glycol precipitation (Maniatis et al., 1982). JC8111 bacterial cells were transformed by electroporation as described (Dower et al., 1988).

The recombinant AAV/TtsA58, AAV/gfp and AAV/CAT-Neo viruses were produced by electroporating 4.0 10⁶ HeLa cells preinfected with adenovirus type 2 (10 pfu/cells, 2 hrs in serum-free medium), with 15 mg of pIM45 and 30 mg of pTR-TtsA58, pTRUF2 or pTR/CAT-Neo, respectively. Electroporation was performed in cytomix medium (120 mM KCl; 0.15 mM CaCl_{2;} 10 mM KH₂PO₄/K₂HPO₄, pH7.6; 25 mM Hepes, pH 7.6; 2 mM EGTA; 5 mM MgCl₂; 2 mM ATP and 5 mM glutathione) at 250 Volt and 1,800 mF. Following pulsing, the cells were diluted into 5 ml of culture medium and cultured in 60 mm tissue dish. After 48hrs, cells were lysed by five cycle of freeze-thawing and then heat-treated for 1 hrs at 56 °C to inactivate adenovirus. Cell debris were eliminated by centrifugation at 1,500 rpm for 5 min and by filtration on a 0.45 mm filter. Stocks were aliquoted and frozen at -20 °C until use. The titer of viral stocks was determined on adenovirus 2-infected HeLa cell monolayers by in situ focus hybridization (Yakobson et al., 1989). Dishes (60 mm diameter) were seeded with 1.0 10⁶ HeLa cells. Twenty-four hours later, the cells were infected with 0.5 ml of medium without serum containing 10⁸ pfu (plaque-forming units) of purified adenovirus, 2.0 10⁸ infectious units (IU) of wild-type AAV and serial dilution of rAAV. After 2 hrs at 37 °C for virus absorption, 5 ml of complete medium were added. Twenty-eight hours post-infection, cells were transferred to nitro-cellulose filters, alkali denatured and hybridised with specific ³²P-DNA probes as described by Yokobson et al. (1989). Foci of cells synthesising recombinant AAV DNA were visualised by autoradiography for 24 hrs at -70 °C. Titers were expressed as rAAV infectious units per millilitre (IU/ml).

CV1 (ATCC), COS-1 (ATCC) and HeLa human cervical carcinoma (ATCC) cells were maintained in high glucose (4.5 g/l). Dubelcco's-modified essential medium with 10% fetal calf serum at 37 °C in 5% carbon dioxide.

AAV/TtsA58 infected HeLa cells were cloned by limit dilution in 96-wells plates (Nunc). Clones originating from single cells were retained and further cultured.

Human pituitary adenoma cells were obtained from a prolactin-secreting tumor removed from the patient by aspiration using a protocol described by O 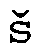 sterom et al. (1984) with slight modifications. The tissue was washed three times in Ca²⁺ and Mg²⁺-free Hank's Balanced Salt Sodium (HBSS) and then cut in small pieces and resuspended in 5 ml of Dispase (grade II, 2.4 U/ml, Boerhinger Mannheim, Germany). Tissue digestion was processed at 37 °C for 1 hrs in a shaking water bath (60 rpm). Non-dissociated tissue was further dispersed with an all-glass Dounce tissue grinder. After centrifugation at 1,500 rpm for 10 min, cell pellet was homogenised in 20 ml of HBSS and filtered on sterile gauze. Cell suspension was layered over Ficoll-isopaque cushions (d = 1.077 g/ml) and centrifuged at 1,000 g for 20 min at 25 °C. The interface fractions were collected and washed twice with HBSS and finally resuspended in culture medium consisting of high glucose Eagle's minimal essential medium with Earle's salts (MEM) supplemented with 1% non-essential aminoacids, 1 mM sodium pyruvate, 2 mM L-glutamine, penicillin, streptomycin, fungizone and 10% fetal calf serum.

Human oligodendroglioma cells were provided by Dr J.Darling (National Hospital, London, UK) and cultured in serum-supplemented medium or in chemically-defined medium as previously described (Tenenbaum et al., 1996).

Cells in culture were washed with medium without serum and then infected with AAV/TtsA58 or AAV/gfp viruses at a multiplicity of 5 IU/cell in a small volume. After two hours at 37 °C for virus adsorption, virus was aspirated and the infected cells were washed and then refed with medium.

SV40 T antigen detection by immunolabeling and in-situ hybridization :
adherent cells were prepared for T antigen detection by seeding overnight on 3 aminopropyl- triethoxy-silane (APES; Sigma)-coated microscope slides (for adherent cells). Non-adherent cells were spread out on APES-coated slides by centrifugation (1000 rpm for 10 min.) using a cytospin centrifuge.

For immunodetection of SV40 T antigen expression, cells were fixed in 30% methanol: 70 % acetone for 10 min at room temperature, rinsed in PBS and further incubated with 1% bovine serum albumin (BSA) in phosphate-buffered saline (PBS : 0.9% NaCl; 0.025% KCl; 0.14% Na₂HPO₄; 0.025% KH₂PO₄, pH 7.4) for 30 min at room temperature. Cells were then incubated with purified L16 monoclonal antibodies against SV40 T antigen (Harlow et al., 1981; commercialised as Ab2, Oncogene Science, Massachussets) at 100 mg/ml overnight at 4 °C. For immunofluorescence, cells were incubated with rabbit antimouse immunoglobulin-fluorescein conjugate (Jackson Immunoresearch Laboratories, West Crove, PA, USA) at a 1:50 dilution in PBS for 1 hrs at room temperature and then embedded in fluorescence-mounting liquid. For immunocytochemistry, cells were incubated with rabbit antimouse immunoglobulin-horseradish peroxidase conjugate (Amersham) at a 1 : 50 dilution in PBS-10% normal goat serum and then reacted with 0.025 mg/ml 3-3-amino-9-ethylcarbazole, 4 mM H₂O₂ in 50 mM acetate buffer pH 4.9 for 10 min to detect antigen-antibody complexes.

For in-situ hybridization, cells were fixed with 4% paraformaldehyde in PBS for 15 min, treated with 0.25% Triton X-100 in PBS for 15 min and washed with PBS. In-situ hybridization were performed as previously described (Delhase et al., 1993; Tenenbaum et al., 1996) using the KpnI-BamHI SV40 fragment or a 1.2 kb fragment of the neomycin gene isolated from pSV2Neo by PCR, labelled with ³⁵S dCTP by random priming (Boerhinger, Mannheim, Germany).

### Southern blot analyses :

High-molecular weight genomic DNA was prepared as described (Maniatis et al., 1982). Low molecular weight DNA was isolated as previously described (Tenenbaum et al., 1993). Samples were treated with DpnI to digest plasmidic (GATC-methylated) DNA. After restriction enzyme digestion, DNA was separated onto a 1% agarose gel in either neutral or- alkaline conditions as described (Maniatis et al., 1982) and transferred to nylon membranes (Hybond N, Amersham) under alkaline conditions. Blots were probed with the 3.0 kb KpnI-BamHI SV40 fragment or a 1.2 kb neo PCR fragment from pSV2neo, labelled with ³²P dCTP by the random priming method.

Cells were seeded on poly L-lysine-coated glass coverslips. Methyl-³H-thymidine (Amersham) was added to the culture medium at 10 mCi/ml. After 2 days at 37 °C, cells were washed three times with PBS, then fixed with 4% paraformaldehyde for 10 min. For autoradiography, the coverslips were processed as previously described (Tenenbaum et al., 1996) and exposed for 4 days.

Fluorescence microscopy was performed with a Zeiss Axiophot microscope equipped with a 450-490 nm excitation filter and Zeiss Plan Neofluar objectives (10X, 20X, 40X).

### Results

### A. Preparation and titration of the AAV/TtsA58 and AAV-gfp recombinant viruses

AAV/TtsA58 recombinant virus was obtained by electroporation of adenovirus preinfected HeLa cells with the pTR-TtsA58 plasmid and pIM45, a plasmid harbouring AAV regulatory (rep) and structural (Cap) genes. AAV/gfp recombinant virus was prepared similarly using pTR-UF2, a plasmid containing the gene coding for the "humanised" gfp under the control of the CMV promoter (Zolotukhin et al., 1996). In these conditions, 30% of HeLa cells were transfected. Two days after electroporation, the cells were lysed by freeze-thawing and adenovirus was heat-inactivated. The titer of the stocks was evaluated by "in situ focus hybridization" (Fig.2; Yakobson et al., 1989). This is a biological assay revealing only functional genomes in the virus preparation. Serial dilutions of the recombinant virus are used to infect HeLa cells in the presence of an excess of wild-type AAV and adenovirus to provide AAV regulatory and structural genes as well as adenoviral helper functions (see Fig.2 A,B). The titers of the stocks varied in the range of 5 10⁵ to 2 10⁶ IU per ml. One IU corresponded roughly to 100 viral rAAV single-stranded DNA molecules. When an excess of AAV/TtsA58 or AAV/gfp virus (10⁵) was used to infect HeLa cells in the absence of adenovirus, no hybridization signal was observed, showing that the recombinant AAV stocks do not contain functional adenovirus after heat inactivation (Fig.2, C).

### B. AAV/TtsA58 transduces HeLa cells 100-fold better than AAV/gfp

Twenty-thousand cells were infected with 10⁵ IU of the AAV/TtsA58 or the AAV/gfp virus stock. When the infected culture was maintained at 33 °C, T antigen was detected by immunocytochemistry in 30-50 % of the cells 3 days after infection (Fig.3A) . As described for SV40-infected cells (Montesano and Lane, 1984), the staining was mainly nuclear. Non-infected cells harboured no background nuclear staining (Fig 3B).

Less than 1% of the cells were transduced by AAV/gfp in the same conditions (Fig.3c & D).

The observed higher transduction efficiency by AAV/TtsA58 as compared to AAV/gfp could be due to the sensitivity of the detection method (immunocytochemistry versus gfp fluorescence). Therefore, the transduction efficiencies of AAV/TtsA58 and AAV/gfp were compared at the level of mRNA expression using in situ hybridization with radioactive probes. After infection of HeLa cells with AAV-TtsA58 at a multiplicity of 5 IU per ml, most of the cells were labelled (Fig.4 A). The input viral DNA, measured 4 hours after infection, did not contribute significantly to the observed signal (Fig. 4 C). The signal was mainly due to the presence of RNA species hybridising to the SV40 probe, as shown by the disappearance of the majority of the labelling after treatment with ribonuclease A (Fig. 4 D). When HeLa cells were infected by AAV/gfp at a multiplicity of 5 IU per ml, about 3 % of the cells were labelled (Fig.4 E) . Thus, using the same number of infectious units, AAV/TtsA58 gave rise to 100 times more transcriptionally active genomes than AAV/gfp. However, about 15 % of the cells were weakly labelled. This labelling could be due either to a low level of mRNA transcripts or to residual input DNA.

To determine whether the presence of an active large T antigen affects the transduction efficiency of the vector, we compared the expression of the mRNA coding for large T antigen at the permissive (33 °C) and non permissive (38 °C) temperature. The same proportion of cells were positive at 33 °C and at 38 °C (Fig.4 A&B), suggesting that the transforming and replication initiation activities of large T which are inactivated at the non permissive temperature, are not responsible for the enhanced transduction efficiency.

### C. Replicative behaviour of AAV/TtsA58 recombinant virus

AAV/TtsA58 virus contains two replication origins: AAV ITRs and SV40 ori. Therefore, the replicative behaviour of the hybrid AAV-SV40 virus was studied.

In the presence of adenovirus and wild-type AAV, AAV/TtsA58 gave rise to the typical AAV-like replicative forms: 3.3 kb linear double-stranded monomers, linear double stranded-dimers and single-stranded DNA (Fig. 5 A). After treatment with PstI, fragments of 2.5 and 0.8 kb resulting from digestion of double-stranded monomers appeared (Fig.1; Fig. 5 B). The replicative intermediates were identical at 33 °C and 38 °C suggesting that the presence of an active T antigen did not affect the replicative behaviour of AAV-TtsA58 in the presence of adenovirus and wild-type AAV.

In the presence of adenovirus but in the absence of wt AAV, AAV/T tsA58 also gave rise to the monomeric and dimeric double-stranded replicative intermediates at a very high efficiency (Fig. 5 C). This was unexpected since the site-specific endonucleasic activity at the terminal resolution site (trs) in the 3' ITR region mediated by Rep proteins, is necessary for a productive replication cycle. Indeed, as already shown by others for other recombinant AAV viruses (Fisher et al., 1996; Ferrari et al., 1996), in the absence of wild-type AAV, co-infection of AAV/gfp with adenovirus gave rise to only a low amount of monomeric replicative forms resulting from the dead-end conversion of input single-stranded DNA to double-stranded DNA

However, AAV/TtsA58 contains a functional SV40 origin. Therefore, it should replicate in monkey (permissive) cells in the presence of an active T antigen. The functionality of both SV40 origin and T antigen present in the pTR-TtsA58 construct was tested in CV1 monkey cells at 33 °C. Plasmidic pTR-TtsA58 DNA was transfected into CV1 ells and DNA replication was evaluated. Figure 5B shows that that of pTR/TtsA58 (lane 6) replicated in CV1 cells with an efficiency comparable to SV40 tsA58 DNA (lane 5) or SV40 tsA58 virus (lane 6). The tsA58 T antigen and the SV40 origin of replication cloned in the AAV vector are thus proficient for replication.

Human cells being semi-permissive for SV40, we tested the possibility that AAV/TtsA58 replicated as an episome in HeLa cells. Low molecular weight, extrachromosomal DNA was isolated 2 and 5 days after infection and sequences containing SV40 origin probed for in Southern blots. No SV40-hybridizing DNA species was observed, except for a slight contamination of input plasmidic DNA present in the viral stock which appeared as DpnI-digested species (Fig. 7 B).

The enhanced transduction efficiency of AAV/TtsA58 is not due to an enhanced second-strand synthesis.

To determine whether the enhanced transduction efficiency by AAV/TtsA58 was due to an enhanced second-strand synthesis, immediately after infection and prior to integration, HeLa cells were infected at 37 °C and incubated for 24 hours at 33 °C. The viral DNA was analysed on alkaline gels in order to better evidence single-stranded DNA (Sambrook et al, 1989). Double-stranded converted genomes harbouring a "turnaround" (closed) end migrate according to the size of a dimer on denaturing gels (Ferrari et al., 1996). Twenty-four hours after infection, a single 3.3 kb band corresponding to the size of the viral DNA but no higher molecular (converted) form appeared (Fig. 6).

### D. AAV/TtsA58 efficiently integrates into the genomic DNA

To determine whether the hybrid AAV/TtsA58 virus integrates into the cellular genome, the high molecular weight (genomic) DNA was extracted, 5 days after infection. The DNA was digested with the PvuII restriction enzyme which cuts twice in the vector (map positions 270 and 3506 in SV40, see Fig. 1). PvuII digestion of pTR-TtsA58 generates a 2.0 kb band corresponding to the internal SV40 sequence as well as a 2.6 kb band and a 3.0 kb band corresponding to the viral insert-pBR322 junctions (Fig. 7A, lanes 4, 5, 6; Fig. 7B, lanes 1, 2, 3). Since no selective pressure was applied, no bias towards particular integration events is expected and the isolated genomic DNA is representative of the actual status of the AAV/TtsA58 DNA in the whole cell population after infection. In these conditions, if integration occurs randomly in the cellular genome, no band corresponding to cell-virus junctions is expected to appear. In contrast, if preferential sites for integration of AAV/TtsA58 exist, bands corresponding to junctions could appear. If integration occurs via the ITRs, a 2.0 kb band, corresponding to the PvuII fragment in the SV40 internal sequence (see Fig. A) is expected, whereas if integration occurs *via an* SV40 sequence, the 2.0 kb band should not appear.

When cells were maintained at 33 °C, a 2.0 kb band, compatible with integration via AAV ITRs, was observed (Fig. 7A, lane 3). Two additional lighter bands appeared, presumably representing integration event(s) at (a) preferential site(s) in part of the cell population. When cells were maintained at 38 °C, a single band was observed (Fig. 7A, lane 2) whose size (about 1.2 kb) was not compatible with integration through AAV ITRs but rather with head-to-tail tandem repeats integrated via an SV40 sequence. Circularisation of the AAV/TtsA58 genome prior to integration via an SV40 sequence could also account for the observed 1.2 kb band.

The number of copies of integrated DNA can be roughly evaluated using plasmidic markers. The observed band corresponded approximately to 1-2 copies per cell. Taking into account that 90 % of the cells were expressing the mRNA coding for T antigen (Fig. 4), this corresponds to 1-2 copies of AAV/TtsA58 per cell.

As expected, the control COS-1 cell line (Fig. 7A, lane 1) gave rise to the 2 kb internal SV40 fragment and to a high molecular weight fragment presumably representing a junction fragment between SV40 and cellular DNA (Gluzman, 1981).

An AAV vector carrying no SV40 sequence (AAV/CAT-Neo) was used as a control to evaluate possible integration at low frequency of rep⁻ AAV vectors in our conditions. HeLa cells were infected with AAV/CAT-Neo virus and the genomic DNA was isolated 5 days after infection and digested with PstI (1 cut in the Neo sequence) or SmaI (2 cuts in AAV ITRs). No band hybridizing to a neo probe was observed, suggesting that in the absence of selective pressure, integration of the AAV/CAT-Neo vector occurs at a frequency too low to be detected in our conditions (Fig. 7C).

To determine which proportion of the cells contained integrated AV/TtsA58 sequences and whether the integrated sequence were stably transmitted to daughter cells, AAV/TtsA58 infected cells were diluted, seeded into 96 wells at a concentration of 1 cell per well, and grown up to visible clones. Single clones were further isolated and 20 µg were digested with PvuII. Five clones out of 9 revealed SV40 hybridizing bands. In all positive clones, a band corresponding to approximately 3 kb appeared (Fig. 7C, lanes 1, 2, 3, 4, 9). In one clone (Fig. 7C, lane 1), an additional band corresponding to approximately 4.0 kb appeared. These bands possibly correspond to junctions between viral and cellular DNA. The 2 kb band corresponding to the PvuII SV40 fragment which appeared in the genomic DNA extracted from uncloned infected cells immediately after infection (Fig. 7A), did not appear after cloning and long-term culturing, suggesting that major rearrangements of the integrated viral sequences occurred.

The clones were infected with Adenovirus and wild-type AAV or with Adenovirus alone. Two days after infection (CPE becomes apparent), low molecular weight DNA corresponding to about 2 10⁵ infected cells was extracted and electrophoresed through a 1 % agarose gel. No band hybridizing with the SV40 probe was observed suggesting that the SV40 sequences still present in the AAV/TtsA58 infected clones after 3 months in culture, were not rescuable and thus presumably did not contain intact ITRs.

### E. AAV/TtsA58 efficiently transduces proliferative and non proliferative human cells

To determine whether the proliferative status affects the transduction efficiency of human cells by AAV/TtsA58, short-term cultures of proliferative, slowly dividing and non-dividing human cells were infected.

Pituitary adenomas cells are semi-adherent cells which can be dissociated from surgical specimen and maintained in culture several days but almost do not divide (Snyder et al., 1978). Prolactinoma cells were infected at a multiplicity of 5 IU per cell, 2 days after dissociation.

After overnight incubation at 37 °C, cells were transferred either at 33 °C or at 38 °C.

Two days after infection, the expression of T antigen was monitored by immunocytochemistry. More than 80% of the infected cells incubated at 33 °C were labelled (fig. 8 A) whereas no staining was observed at 38 °C (Fig.8 B) or in the non infected culture at 33 °C (not shown). The cells were passaged weekly by slight trypsinisation (to eliminate adherent contaminating fibroblasts and folliculostellate cells). They could be maintained up to 10 weeks but the cell number decreased at each passage. After 6 weeks (passage 6), the majority of the cells still expressed T antigen at 33 °C (Fig.8, C and D).

IN1574 oligodendroglioma cells were previously described (Tenenbaum et al., 1996). Briefly, when these cells were cultured in serum-supplemented medium, they proliferated and expressed the GFAP astrocytic marker. When they were cultured in serum-free insulinsupplemented medium, they did not divide and expressed the O1 oligodendrocyte marker. Few cells (2-3%) were still dividing 24 hrs after removal of the serum, but no cell divided from the second day on. IN1574 cells cultured in serum-supplemented medium were infected with AAV/TtsA58 at a multiplicity of about 5 I.U. per cell. Most cells divided within 48 hrs after seeding (Fig.8 E, F). Infection was performed 24 hrs after seeding. T antigen was expressed in about 30% of infected cells 3 days after infection. The specificity of the labelling was demonstrated by the absence of staining in non infected cells (Fig.8 F).

IN1574 cells cultured in serum-free medium were infected 3 days after seeding. No ³H-thymidine labelled cell appeared between day 2 and day 4 after seeding (Fig. 8, G and H). T antigen was expressed in about 10% of infected cells, 3 days after infection (Fig 3, G). No nuclear labelling was observed when cells were maintained at 38 °C (Fig. 3 H).

An hybrid AAV-SV40 virus consisting in AAV ITRs fused to the early operon of the SV40tsA58 virus is described. Upon coinfection with adenovirus and wild-type AAV, this virus gave rise to the typical AAV replication intermediates: double-stranded monomeric and dimeric replication forms and single-stranded DNA. Upon co-infection with adenovirus only, AAV/TtsA58 also gave rise to all AAV-like replicative forms. In the absence of helper virus, no extrachromosomal DNA appeared and the hybrid virus was found integrated at a high frequency in the cellular genome. At 33 °C (active T antigen) integration occurred via the ITRs. Two bands presumably corresponding to cellular-viral junctions were observed in a mixed (uncloned) population of infected cells, suggesting that (a) preferential site(s) for integration of AAV/TtsA58 may exist in the human genome. At 38 °C (inactivated T antigen), integration occurred via an internal SV40 sequence. Immunocytochemistry revealed an unusually high transduction efficiency at 33 °C; more than 50% of infected HeLa cells expressed T antigen 2 days after infection with AAV/TtsA58 at a multiplicity of 5 infectious units per cell. Using an AAV vector carrying the "humanised" gfp and the neo reporter genes (AAV/gfp), less the 1% of the cells expressed gfp in the same conditions. Confirming these data, in situ hybridization showed that almost 100% of the infected HeLa cells expressed the mRNA for large T antigen after infection with AAV/TtsA58 at a multiplicity of 5 infectious units per ml whereas only 3 % of the cells efficiently expressed the neo mRNA after infection with AAV/gfp.

Previous studies with different reporter genes already showed that transduction efficiencies by rAAV are low (Samulski et al., 1989; Chiorini et al., 1995; Fisher et al., 1996; Ferrari et al., 1996). The transduction efficiency could be drastically enhanced by coinfection with adenovirus (Fisher et al., 1996; Ferrari et al., 1996) or by treatments which arrest cells in the S phase (Ferrari et al., 1996). Second-strand synthesis triggered by adenovirus E4 and El gene products and by UV has been correlated with this enhancement of transduction efficiency by AAV vectors (Fisher et al., 1996, Ferrari et al., 1996). The vector was found in an episomal transcriptionally active double-stranded form. No evidence for integration has been given.

Integration of helper-free rep⁻ recombinant AAV was never clearly demonstrated except when selection was applied (Samulski et al., 1989) and in the cases in which vectors integrated, it was not in chromosome 19 (review: Flotte and Carter, 1995). In vivo, integration was never demonstrated and in one case in which the status of the vector was looked for at long-term after infection, it was found in an extrachromosomic form (Afione et al. 1996). Interaction between Rep and AAV ITRs on one hand and the S1 integration site on chromosome 19 on the other hand, points to a direct implication of the Rep proteins in the integration process (Weitzman et al., 1994).

The high transduction efficiency of AAV/TtsA58 was not due to an enhanced conversion of input DNA to double-stranded DNA species but AAV/TtsA58 DNA was found to be associated to cellular genomic DNA at high frequency. Let us notice that since no antibiotics selection was applied to the infected cultures, the high frequency of integration reflects the actual status of viral DNA in the culture shortly after infection.

This is the first report of integration of an AAV rep- vector in the absence of selection pressure.

In the presence of adenovirus and in the absence of AAV Rep proteins, AAV/TtsA58 gave rise to a burst of replication intermediates both monomeric and dimeric. According to the current model for AAV replication, this can occur only in the presence of Rep proteins which resolve the "turn-around" replicative intermediates by recognising and cleaving the terminal resolution site (trs) (Berns and Bohenzky, 1987). Our data thus suggest that large T can replace Rep for the site-specific endonucleolytic cleavage at the terminal resolution site (trs). Consistent with this hypothesis, Ward and Berns (1989) showed that an AAV/SV40 hybrid plasmid carrying the SV40 replication origin can be excised from the pBR322 backbone by adding T antigen in an in vitro replication assay. The homology between Rep and large T proteins (Anton and Lane, 1986, Astell et al., 1987), further suggests a possible interaction between T antigen and AAV ITRs. Although the hybrid virus integrated efficiently at both - temperatures, the mechanism of integration was different. At 33 °C it integrates via AAV ITRs whereas at 38 °C, the analysis of the genomic DNA revealed a fragment consistent with integration via an internal (SV40) sequence and involving AAV head-to-tail tandem repeats. Consistently, former studies on the structure of integrated wild-type AAV (Cheung et al., 1980, McLaughlin et al., 1988) and recombinant AAV genomes (Samulski et al., 1989) had suggested that viral insertions are usually in a tandem head-to-tail orientation. Alternatively, AAVtsA58 could circularise prior to integration in HeLa cells and integrate either via AAV ITRs (generating a 2 kb PvuII band) or via an SV40 internal sequence (generating a 1.4 kb PvuII band). This hypothesis is supported by our data (Tenenbaum and Winocour, 1989) showing that an AAV recombinant virus carrying SV40 origin (Labow et al., 1987) replicates as a circular molecule in monkey (permissive) cells.

Simian virus 40 (SV40) infection in monkey cells follows a lytic cycle but in other cells, including human cells, the infection is abortive and the SV40 genome can become integrated into the host cell genome resulting in transformation of the cell. The large T antigen is required for both replication and transformation. It contains several functional domains, which have been related to distinct biochemical functions, including DNA-binding, ATPase, DNA helicase, binding to cellular proteins as p53, pRb and DNA polymerase a, nuclear localisation. (Manfredi and Prives, 1994). A wide range of mutants have been identified which have clearly shown that the replication and transformation functions of T antigen can be genetically separated (Kalderon and Smith, 1984). The tsA58 thermosensitive mutant is defective for both replication initiation (Reynisdottir et al., 1990) and transformation (Radna et al., 1989) at high (non permissive) temperatures. However, at the biochemical level, it retains some functions as DNA helicase and replication elongation (Ray et al., 1992).

The high transduction efficiency was observed at both permissive and non permissive temperatures as shown by the high proportion of cells expressing the mRNA coding for T antigen. Furthermore, a productive AAV-like replication was observed in the absence of wild-type AAV (and thus Rep proteins) at both temperatures. Integration efficiencies were also comparable at both temperature. This suggest that (a) function(s) that is retained by the tsA58 mutant at high temperature is responsible for the resolution of AAV replication intermediates and stimulation of integration (both of which could be responsible for the high transduction efficiency). The tsA58 mutation is located in the second half of the large T antigen in a region involved in binding with p53 (Manfredi and Prives, 1994).The stability of tsA58 T protein is the same at both temperatures (Reynisdottir et al., 1990). However, the main biological functions of the tsA58 T antigen (immortalisation, replication initiation) are abolished at the non-permissive temperature (refs). Impaired enzymatic activities involve formation of oligomers, binding to p53, association with nuclear matrix, association with polymerase a, binding to SV40 origin of replication. However, some activities are conserved: elongation of viral DNA replication and DNA helicase (Ray et al., 1992). This last enzymatic activity could be responsible for a more efficient recombination between single-stranded viral DNA and cellular DNA. Consistently, the RecA protein which harbours homology with both Rep and T antigen (Astell et al. 1987; Anton and Lane, 1986) in the helicase domain of the protein, catalyses recombination in E.coli (Seif, 1982).

However, the different mechanism of integration at 33 °C and 38 °C suggests that a function that is inactivated at high temperature in the tsA58 mutant is responsible for an interaction between AAV ITRs and the cellular genome which leads to integration via the ITRs.

The AAV/TsA58 vector provided efficient expression of SV40 T antigen in dividing and non-dividing human cells: pituitary adenomas, oligodendrogliomas and in the HeLa cell line. Long-term expression was demonstrated in prolactinoma cells. In oligodendroglioma cells however, no expression was detected one month after infection. This can be attributed to several reasons: inactivation of the SV40 promoter driving the expression of T antigen in these cells; loss of the viral sequences; death of the infected cells.

The properties of the AAV/TtsA58 vector could be exploited for the development of efficient, integrative gene therapy vectors. However, the 3.0 kb SV40 fragment present in the vector drastically limits the size of the insert that can be still cloned between the ITRs in order to obtain a vector that can still be encapsidated. Indeed, AAV vectors larger than the wild-type virus (4.7 kb) are poorly or not encapsidated (Hermonat and Muzyczka, 1984). Furthermore, given the cell-transforming activity of the large T antigen, the introduction in a gene therapy vector of a complete functional early SV40 operon is not conceivable. The high transduction efficiency by AAV/TtsA58 at the non-permissive temperature, seems to indicate that only some functions (i.e. replication elongation and DNA helicase) and thus only some functional domains of the large T antigen are necessary. A wide range of mutants both deletion and missense, have been identified which have clearly shown that the replication and transformation functions of SV40 large T antigen can be genetically separated, e.g. replication-proficient, transformation-deficient deletion mutants have been described (Kalderon and Smith, 1989). The minimal SV40 fragment required to observe the transduction enhancing effect is currently being determined. Hopefully, the T (or t) antigen function(s) which are responsible for the enhancement of transduction by rAAV will be identified and introduced into gene therapy constructs.

AAV/TtsA58 alleviates some of the limitations encountered in previous attempts to transform human cells with SV40 T antigen. Indeed, the low frequency of integration of SV40 virus and the poor transfection efficiency of many cell types by T antigen-expressing plasmids suggest that the failure to immortalise human cells resides in limitations imposed by the introduction and expression of T antigen rather than on the mechanism of immortalisation of these cells. Retroviral vectors expressing T antigen have already been developed but their tissue specificity is limited and they can not infect post-mitotic cells. AAV/TtsA58 vector providing an optimal transduction system for a wide variety of human cells (including non-dividing cells) is a potential immortalising vector with promising properties. It was shown in this study that AAV/TtsA58 efficiently integrates into the genome of dividing cells. Whether it integrates into non dividing cells was not looked for, due to the paucity of material available. However, the high transduction efficiency of non-dividing adenoma cells by AAV/TtsA58 and the long-term expression of T antigen in these cells suggest that the vector behaves in the same way as in proliferative cells.

### REFERENCES

1. Afione, S.A., Conrad, C.K., Kearns, W.G., Chunduru, S., Adams, R., Reynolds, T.C., Guggino, W.B., Cutting, G.R., Carter, B.J., Flotte, T.R. 1996. In vivo model of adeno-associated virus vector persistence and rescue. J.Virol. 70: 3235-3241.
2. Alexander, I.E., Russel, D.W., Spence, A.M., and Miller, A.D. 1996. Effects of gamma irradiation on the transduction of dividing and nodividing cells in brain and muscle of rats by adeno-associated virus vectors. Hum. Gene Ther. 7: 841-850.
3. Ali R.A. Reichel, M.B., Trasher, A.J., Levinsky, R.J., Kinnon, C., Kanuga, N., Hunt, D.M. and Bhattacharya, S.S. 1996.Gene transfer into the mouse retina mediated by an adeno-associated viral vector. Human Molecular Genetics. 5, 591-194.
4. Anton, I.A. and Lane, D.P. 1986. Non-structural protein 1 of parvoviruses: homology to purine nucleotide using proteins and early proteins of papovaviruses. Nucl.Ac.Res. 19: 7813.
5. Astell C.R., Mol, C.D. and Anderson, W.F. 1987. Structural and functional homology of parvovirus and papovavirus polypeptides. J.Gen.Virol. 68: 885-893.
6. Berns, K.I. and Bohenzky, R.A., 1987. Adeno-associated viruses: an update. Adv. Virus Res. 32: 243-306
7. Boissy, R. and Astell, C.R., 1985. Escherichia coli recBC sbcB recF hosts permits the deletion resistant propagation of plasmid clones containig the 5'-terminal palindrome of minute virus of mice. Gene 35, 179-185.
8. Cheung, A., Hoggan, M.D., Hauswirth, W.W and Berns, K.I. (1980). Integration of the adeno-associated virus genome into cellular DNA in latently infected Detroit 6 cells. J.Virol. 33, 739-804.
9. Chiorini, J.A., Wendtner, C.M., Urcelay, E., Safer, B., Hallek, M. and Kotin, R.M. 1995. High-efficiency Transfer of the T cell co-stimulatory molecule B7-2 to lymphoid cells using high-titer recombinant adeno-associated virus vectors. Hum. Gene Ther. 6: 1531-1541.
10. Delhase, M., Vergani, P., Malur, A., Velkeniers, B., Teugels, E., Trouillas, J., and Hooghe-Peters, E.L. 1993. Pit1/GHF-1 expression in pituitary adenomas: further analogy between human adenomas and rat SMtTW tumours. J.Mol.Endocrinol. 11: 129-139.
11. Dower et al. 1988. Nucl. Ac. Res. 16, 6127-6145.
12. Fisher, K.J. Gao, G.-P., Weitzmann, M.D., DeMatteo,R., Burda, J.F., and Wilson, J.M. 1996. Transduction with recombinant adeno-associated virus for gene therapy is limited by leading-strand synthesis. J.Virol. 70: 520-532.
13. Ferrari, F.K., Samulski, T., Shenk, T. and Samulski, R.J. 1996. Second-strand synthesis is a rate-limiting step for efficient transduction by recombinant adeno-associated virus vectors. J.Virol. 70: 3227-3234.
14. Flotte, T.R. and B.J. Carter. 1995. AAV vectors for gene therapy. Gene Therapy 2: 357-362.
15. Gluzman, Y. 1981. SV40-transformed simian cells support the replication of early SV40 mutants. Cell, 23: 175-182.
16. Halbert, C.L., Alexander, I.E., Wolgamot, G.M. 1995. Adeno-associated virus vectors transduce primary cells much less efficiently than immortalized cells. J.Virol. 69: 1473-1479.
17. Harlow, E., Crawford, L.V., David, C.P. and Williamson, N.M. 1981. Monoclonal antibodies specific for simian virus 40 tumor antigens. J.Virol. 39, 861-869.
18. Hermonat, P.L. and Muzyczka, N. 1984. Use of adeno-associated virus as a mammalian DNA cloning vector: transduction of neomycin resistance into mammalian tissue culture cells. Proc.Natl.Acad.Sci.USA 81: 6466-6470.
19. Kaplitt, M.G., Leone, P., Samulski, R.J., Xiao,X., Pfaff,D.W., O'Malley, K.L. and During, M.J. 1994. Long-term gene expression and phenotype correction using adeno-associated virus vectors in the mammalian brain. Nature Genet.8: 148-154.
20. Kalderon, D. and Smith, A.E. (1984) In vitro mutagenesis of a putative DNA binding dopain of SV40 large T. Virology 139, 109-137.
21. Kotin, R. M., M. Siniscalco, R.J. Samulski, X. Zhu, L. Hunter, C.A. Laughlin, S. McLaughlin, N. Muzyczka, M. Rocchi, K.I. Berns. 1990. Site-specific integration by adeno-associated virus. Proc Natl Acad Sci. USA 87: 221-2215.
22. Labow, M.A., and Berns, K.I. 1988. The adeno-associated virus rep gene inhibits replication of an adeno-associated virus/simian virus 40 hybrid genome in cos-7 cells. J.Virol. 62: 1705-1712.
23. Labow, M.A., Hermonat, P.L. and Berns, K.I. 1986. Positive and negative autoregulation of the adeno-associated virus type 2 genome. J.Virol. 60: 515-524.
24. Labow, M.A., Graf, L.H. and Berns, K.I. 1987. Adeno-associated virus gene expression inhibits cellular transformation by heterologous genes. Mol. Cel. Biol. 7: 1320-1325.
25. Manfredi J.J, and Prives, C. 1994. The transforming activity of simian virus 40 large tumor antigen. Biochimica et Biophysica Acta 1198: 65-83.
26. Maniatis, T., Fritsch, E.F. and Sambrook, J. 1982. Molecular cloning: a laboratory manual. Cold Spring Harbor, N.Y.
27. McCarty, D.M., Christensen,M. and Muzyczka, N.. 1991. Sequences required for the coordinate induction of the AAV p19 and p40 propoters by the Rep protein. J.Virol. 68: 4988-4997.

### A. Labow et al. 1987 d13-5/SV

28. McLaughlin, S.K., Collis, P., Hermonat, P.L., and Muzyczka, N. 1988. Adeno-associated virus general transduction vectors: analysis of proviral structures. J.Virol. 62: 1963--1973.
29. Mendelson, E., Smith,M/G/, Miller, I.L. and Carter, B.J. 1988. Effect of a viral rep gene on transformation of cells by an adeno-associated virus vector. Virology 166: 612-615.
30. Montesano, X. and Lane, D.P. 1984. Monoclonal antibody to simian virus small t. J.Virol. 51, 760-767.
31. Oosterom, R., Blaauw,G., Singh,R., Verleun,T., and S.W.J. Lamberts. 1984. Isolation of large numbers of dispersed human pituitary adenoma cells by aspiration. J.Endocrinol. Invest. 7: 307-311.
32. Podsakoff,G., Wong,K.K, and Chaterjee,S. 1994. Efficient gene transfer into nondividing cells by adeno-associated virus based vectors. J.Virol. 68, 5656-5666.
33. Radna, R.L.,Caton, Y., Jha, K.K., Kaplan, P., LI, G., Traganos, F. and Ozer, H.L. 1989.Growth of immortal simian virus 40 tsA-transformed human fibroblasts is temperature-dependent. Mol.Cell.Biol. 9, 3093-3096.
34. Rafferty, K.A., Ruben, R.L., and Young, S.K. 1978. A virus-producing cell line developped by transformation of human parapharyngeal cells. In vitro, 14: 227-235.
35. Ray, S., Anderson, M.E., Loeber, G., McVey, D. and Tegtmeyer, P. 1992. Functional characterization of temperature-sensitive mutants of simian virus large T antigen. J.Virol. 66: 6509-6516.
36. Reynisdottir I, O'Reilly, D.R, Miller, L.K. and Prives, C. 1990. Thermally inactivated simian virus 40 tsA58 mutant T antigen cannot initiate viral DNA replication in vitro. J.Virol. 64: 6234-6245.
37. Russel, D.W., Miller, A.D. and Alexander, I.E. 1994. Adeno-associated virus vectors preferentially transduce cells in the S phase. Proc.Natl.Acad.Sci.USA 91: 8915-8919.
38. Russel, D.W., Alexander, I.E., and Miller, A.D. 1995. DNA synthesis and topoisomerase inhibitors increase transduction by adeno-associated virus vectors. Proc.Natl.Acad.Sci.USA 92: 5719-5723.
39. Samulski, R.J., 1993. Adeno-associated virus: integration at a specific locus. Curr. Opin. Genet. Dev. 3: 74-80.
40. Samulski, R.J., Chang, L.S. and Shenk, T. 1989. Helper-free stocks of recombinant adeno-associated viruses: Normal integration does not require viral gene expression. J.Virol. 63, 3822-3828.
41. Seif, R. 1982. New properties of simian virus large T antigen. Mol. Cell. Biol. 2, 1463-1471.
42. Shelling,A. and Smith, M.G. 1994. Targeted integration of tr,asfected and infected adeno-associated virus vectors containing the neomycin resistance gene. Gene Ther. 1: 165-169.
43. Shleh 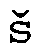 fer, J.R., Ehrlar, M., and zur Hausen, H. 1986. Vaccinia virus, herpes simplex virus, and carcinogens induce DNA amplification in a human cell line and support replication of a helper virus dependent parvovirus. Virology 152: 110-117.
44. Schirmbeck, R. and Deppert, W. 1987. Specific interaction of simian Virus 40 large T antigen with cellular chromatin and nuclear matrix during the course of infection. J.Virol. 61: 3561-3569.
45. L.Tenenbaum, F.Dupont, P.Spegelaere, L.Zentilin, P.Norio, M.Giacca, S.Riva, A.Falaschi and J.Rommelaere. 1993. Inhibition of heterologous DNA replication by the MVMp non-structural NS-1 protein : Identification of a target sequence. Virology: 197, 630-641.
46. Tenenbaum, L. Teugels, E. Dogusan, Avellana-Adalid, V. Z. and Hooghe-Peters, E.L. 1996. Plastic phenotype of human oligodendroglial tumour cells in vitro. Neuropathol. Appl. Neurobiol. 22:
47. Tratschin, J.D., Miller, I.L., Smith, M.G. and Carter, B.J. (1985). Adeno-associated virus vector for high frequency integration, expression, and rescue of genes in mammalian cells. Mol. Cel. Biol. 5 : 3251-3260.
48. Weitzmann, M.D., Kyöstio, S.R.M., Kotin, R.M., and Owens, R.A., 1994. Adeno-associated virus (AAV) Rep proteins mdiate complex fromation between AAV DNA and its integration site in human DNA. Proc. Natl. Acad. Sci. USA 91, 5808-5812.
49. Yakobson, B., Koch, T. and E. Winocour. 1987. Replication of adeno-associated virus in synchronized cells without the addition of a helper virus. J. Virol. 61 : 972-981.
50. Yakobson, B., Hrynko, T.A., Peak, M.J. and E. Winocour. 1989. Replication of adeno-associated virus in cells irradiated with UV light at 254 nm. J. Virol. 63, 1023-1030.
51. Yalkinoglu, A.O., Heilbronn, R., Shlehöfer, J.R. Ehrlar, M., and zur Hausen, H. 1986. DNA amplification of adeno-associated virus as a response to genotoxic stress. Cancer Res. 48 : 3123-3129.
52. Zhou, S.Z., Cooper, S., Kang, L.Y., Ruggieri, L., Heimfeld, S., Srivastava, A. and Broxmeyer, H.E. 1994. Adeno-associated virus 2-mediated high efficiency transfer into immature and mature subsets of hematopoietic progenitor cells in human umbilical cord blood. J. Exp. Med. 179 : 1867-1875.

## Claims

1. A method for increasing the efficiency of the integration of a parvoviral vector into a cell genome and the expression of genetic sequence carried by said vector, wherein this increase results from the insertion or the addition of non-parvoviral cis- and/or trans-elements made of nucleic acid sequence(s) and/or amino acid sequence(s) (encoded by said sequence(s)), into a parvoviral vector.

2. The method of claim 1, wherein said parvoviral vector is derived from human, mammal or avian virus belonging to the category of adeno-associated viruses (AAV).

3. The method of claim 1 wherein said parvoviral vector is derived from human or mammal autonomous parvoviruses, preferably the parvovirus H1, the parvovirus Minute of Mice (MVMp or MVMi) or the parvovirus LuIII.

4. The method of claim 1 to 3, wherein said parvoviral vector is limited to its palindromic extremities, a portion thereof, or a mutant thereof having the same functionality.

5. The method according to any of the claims 1 to 4, wherein said cis- and/or trans-elements correspond to sequence(s) with transcription-activating function(s), deriving from prokaryotic, eukaryotic or viral genomes.

6. The method according to any of the claims 1 to 5, wherein said cis- and/or trans-elements correspond to sequence(s) with recombination- or integration-promoting function(s), deriving from prokaryotic, eukaryotic or viral genomes.

7. The method according to any of the claims 1 to 6, wherein said cis- and/or trans-elements contain a nuclear localisation signal.

8. The method according to any of the claims 1 to 7, wherein said cis- and/or trans-elements derive from a virus of the papovirus family, preferably the SV40 virus.

9. The method according to any of the claims 1 to 8, wherein said cis- and/or trans-elements derive from the SV40 early operon, a portion thereof or a mutant thereof.

10. The method according to claim 9, wherein said cis- and/or trans-elements are limited to (some) sequence(s) of the SV40 early operon encoding one or more of the following functions : replication elongation, DNA helicase, transcriptional activation and nuclear localisation.

11. The method according to claim 9 or 10, wherein said cis- and/or trans-elements derive from the SV40 early operon which lacks part(s) or all the nucleotide and/or amino acid sequence(s) encoding the amino acids 105 to 115 of the large T antigen.

12. A vector obtained by to any of the method according to claims 1 to 11.

13. A parvoviral vector comprising insertion or addition of non-parvoviral cis- and/or trans-elements nucleic acid and/or amino acid sequence(s) of the SV40 early operon which do not comprise one or more sequence(s) corresponding to the following functions : replication elongation, DNA helicase, transcriptional activation and nuclear localisation.

14. A vector according to claim 13, which lacks part(s) or all the nucleotide and/or amino acid sequence(s) encoding the amino acids 105 to 115 of the large T antigen.

15. A vector according to claims 12 to 14, which is derived from human, mammal or avian virus belonging to the category of adeno-associated viruses (AAV).

16. A vector according to claim 15, wherein said vector is derived from human or mammal autonomous parvoviruses, preferably the parvovirus H1, the parvovirus Minute of Mice (MVMp or MVMi) or the parvovirus LuIII.

17. A cell genetically transformed by the vector according to any of the claims 12 to 16.

18. A cell according to claim 17, being a cell chosen from the group consisting of glial, neuronal, fibroblastic or pituitary, hematopoietic (including stem cells) origin.

19. Pharmaceutical composition comprising the vector according to any of the claims 12 to 16 or the cell according to the claim 17 or 18 and a pharmaceutically acceptable carrier.

20. The use of the vector according to any of the claims 12 to 16, for the immortalisation of non human cells or human somatic cells which are preferably selected from the group consisting of cells of the endocrine, neuronal or immune system.

21. The use of the vector according to any of the claims 12 to 16, for the transfer of genetic sequence into dividing and non-dividing cell.

22. The use of the pharmaceutical composition according to claim 19, for the preparation of a medicament in the treatment and/or the prevention of diseases, including human genetic disorders, cancers and neurodegenerative diseases.

23. Non-human animal, preferably a non-human mammal, genetically modified by the vector according to any of the claims 12 to 16 or by the cell according to any of the claims 17 or 18.
